# EUROPÄISCHE PATENTANMELDUNG

(11) **EP 1 927 334 A1**
(43) Veröffentlichungstag der Anmeldung: **04.06.2008**
(21) Anmeldenummer: 07021671.8
(22) Anmeldetag: 08.11.2007
(51) Int. Cl.: A61K 8/02, A61K 8/21, A61K 8/24, A61Q 11/00

(54) **Kits, deren Herstellung und Verfahren zur aufhellenden Beschichtung von Zähnen**

(30) Priorität: 21.11.2006 DE 102006055224
(71) Anmelder: Heraeus Kulzer GmbH, 63450 Hanau (DE)
(72) Erfinder: Busch, Susanne Dr., 61267 Neu Anspach (DE); Hoffmann, Marcus Dr., 61250 Usingen (DE); Utterodt, Andreas Dr., 61267 Neu Anspach (DE); Mätzig, Christoph Dr., 63526 Erlensee (DE)
(74) Vertreter: Kühn, Hans-Christian

(57) **Zusammenfassung**

Es wird ein Verfahren zur Aufhellung von Zähnen beschrieben, unter Verwendung eines Kits mit den Komponenten
**A** eine Gelbildner enthakende, alkalische, wässrige Natriumfluoridlösung,
**B** ein getrocknetes, in Filmstreifen vorliegendes Gel enthaltend
**•** B1 mindestens einen Gelbildner,
**•** B2 Phosphat- oder Hydrogenphosphationen,
**•** B3 optional mindestens eine Aminosäure,
**•** B4 optional Fluorid,
**•** B5 optional Glycerin,
**•** B6 optional eine Carbonsäure oder ein Puffersystem für einen der pH-Werte von 4 bis 7.

**C** ein getrocknetes, in Filmstreifen vorliegendes Gel enthaltend
• C1 mindestens einen Gelbildner,
• C2 Calcium-Ionen,
• C3 optional Glycerin,

sowie die Herstellung des Kits.

## Beschreibung

Die Erfindung betrifft Kits zur aufhellenden Beschichtung von Zähnen, die Herstellung der Kits und diese verwendende Verfahren zur aufhellenden Beschichtung von Zähnen.

Glänzende, strahlend weiße Zähne gelten als gesund und besitzen in der Gesellschaft einen hohen Stellenwert. Allerdings fördern die angebotenen Produkte zur Zahnaufhellung die tatsächliche Gesundheit der Zähne nicht. Die Zähne können spröde und rissig werden und ihre natürliche Opaleszenz einbüßen. Die moderne Ernährung fördert die Erosion von Zahnsubstanz, so dass auch ohne das Auftreten von Karies die natürliche Schmelzschicht zunehmend dünner wird und die gelblich opake Farbe des Dentins durchschimmert.

Zur Aufhellung von Zähnen werden vorwiegend Bleichsysteme eingesetzt, die mittels starken Oxidationsmitteln wirken. Die Konzentrationen liegen je nach Applikationsform zwischen 10 -35 % Peroxid. Insbesondere werden konz. Wasserstoffperoxidlösungen oder Carbamidperoxid eingesetzt. Die Wirkungsweise beruht auf der oxidativen Entfärbung von eingelagerten organischen Farbstoffen. Sie haben eine aggressive Wirkung auf die Mundschleimhaut, so dass der Kontakt unbedingt vermieden werden muss. Starke Oxidationsmittel könnten jedoch auch strukturrelevante Proteine im Schmelz degradieren. Der Gehalt an natürlichen Makromolekülen im Zahnschmelz ist abhängig vom Reifungsgrad und beträgt beim Erwachsenen ca. 1-2 Gew %. Proteine finden sich bevorzugt auf der Oberfläche der schmelzprismatischen Einheiten, können aber auch in der Kristallstruktur integriert sein. Kennzeichen der Biomineralisation sind Kompositsysteme aus anorganischem Mineral und organischen Komponenten (bevorzugt Proteine und Polysaccharide). Das spezifische Zusammenspiel führt zu erhöhter Bruchfestigkeit. Wird die organische Komponente chemisch entfernt, ist mit einer Versprödung zu rechnen. Nach der oxidativen Zahnaufhellung kann erwartet werden, dass die Zugänglichkeit für Verunreinigungen zumindest in den ersten Tagen nach der Behandlung erhöht und die Schmerzempfindlichkeit eventuell stark erhöht ist. Vermutlich wird durch die Oxidation der biologischen Matrix ein feines Porensystem durchgängig. Dadurch können Schmerzreize leichter an den Zahnnerv geleitet werden und unerwünschte Fremdstoffe besser ein diffundieren. Manche Bleich-Systeme arbeiten bei niedrigen pH-Werten, was darüber hinaus zu einer Demineralisation führen kann und eine zusätzliche Schwächung für den Zahn bedeutet. Topographische Untersuchungen an gebleichten Humanzähnen zeigen eine wahrnehmbare Zunahme der natürlichen Porosität (see Bitter N. C. and Sanders J., L.: The effect of four bleaching agents on the enamel surface: A scanning electron microscopic study. Quintessence Int 1993, 24: 817-24). Daraus resultiert eine erhöhte Empfindlichkeit gegenüber Säureangriffen. Obwohl die Härte des Zahns nach der Bleichprozedur keine messbare Verminderung zeigt, belegen Studien an gebleachten Zähnen eine deutliche Verminderung der Mikrohärte und verstärkte Auflösungserscheinungen nach simulierten Demineralisations-Remineralisationszyklen. Der Effekt wird zwar durch die Gabe von Fluorid etwas vermindert, aber die Schwächung wird nicht aufgehoben (see Attin T., Kocabiyik M., Buchalla W., Hanning C., Becker K.: Susceptibility of Enamel Surfaces to Demineralisation after Application of Fluoridated Carbamide Peroxide Gels, Caries Res 2003: 37: 93-99). Eine andere Studie belegt, dass die Zug- und Bruchfestigkeit von Schmelz nach Einwirkung von Bleichlösungen um bis zu 30% abnimmt (see: Cavalli, V. et al: Effect of carbamide peroxide bleaching agents on tensile strength of human enamel. Dental Materials (2004) 20, 733-9). Eine Schmerzstudie belegt, dass nach einer Behandlung mit 35% Wasserstoffperoxidlösung 2/3 der Patienten über mäßige bis starke Schmerzen berichteten, die bis zu 48 Stunden nach der Behandlung andauerten (see Nathanson D, Parra C.: Bleaching vital teeth: a review and clinical study. Compend. Contin. Educ. Dent. 1987; 8(7): 490-7). Die Studien ergaben zwar keine eindeutigen Hinweise auf eine irreversible Pulpa-Schädigung, im Tierversuch traten aber nach dem Bleichen von vitalen Hundezähnen Fälle von massiven Entzündungen, Dentinresorption bis hin zum Absterben der Pulpa auf (see: Nathanson, D.: Vital Tooth bleaching: Sensitivity and Pulpal considerations, JADA, 128 (April 1997) 41-4). Vorraussetzung für die konventionelle Bleich-Prozedur sind in jedem Fall gesunde Zähne. Hypersensible Zähne, freiliegende Zahnhälse oder Karies gelten als Kontraindikation. Denkbar ist allerdings, dass sich auch bei gesunden Zähnen Schäden durch wiederholtes Bleachen akkumulieren.

Einige Darreichungsformen, die als geeignet beschrieben werden, die Mineralisation von Apatit auf der Zahnoberfläche anzuregen, sind bekannt. US 6,521,251 beschreibt eine Zusammensetzung, die neben Carbamidperoxid Kalziumphosphate enthält, deren Löslichkeit etwas besser ist, als die von Apatit, wie Mono-Di-oder Trikalziumphosphat. Dennoch sind all diese Kalziumphosphate nur wenig wasserlöslich, daher ist eher eine abrasive als eine remineralisierende Wirkung zu erwarten. Tatsächlich beschreibt US 5,851,514 unter anderem die Zugabe von Dikalziumphosphat als Abrasivum.

US 6,419,905 erwähnt die Zugabe von Kaliumsalzen (z. B. Phosphate) und Fluorid zum Peroxid. Fluorid ist geeignet, aus dem Speichel Kalzium- und Phosphat-lonen zu binden, wodurch Fluorapatit (FAP) ausfällt. Wenn keine weiteren Ionen zugefügt werden, wird auch die Bildung von CaF₂ beobachtet. Die Kalziumfluoridpartikel können in der Plaque gespeichert werden und über längere Zeit Fluorid abgeben, da sie etwas löslicher sind, als Apatit. Jedoch werden vor einem professionellen Bleichen alle Zähne durch intensive Reinigung von der Plaque befreit. JP 20000051804 beschreibt den parallelen Einsatz von konzentrierter Phosphorsäure, konz. H₂O₂ und Fluorapatitpulver. Problematisch erscheint hier der Einsatz der konzentrierten Phosphorsäure, die gesunden Zahnschmelz merklich auflösen kann. Zudem ist die Bleichlösung stark ätzend und darf nicht in Kontakt mit dem Zahnfleisch kommen, was aber in abgeschwächter Form für alle oxidativ wirkenden Zahnaufheller gilt. Zudem führt die wiederholte Anwendung nicht zu einer Zunahme der Remineralisationsschicht. Es wird vermutet, dass sich hier allenfalls aufgelöster Zahnschmelz wieder niederschlägt. Eine säurefreie Anwendung wird in US 20050281759 beschrieben. Als wesentlicher Inhaltsstoff wird hier Kalziumperoxophosphat vorgeschlagen. Kerngedanke ist hier, dass eine einzige Substanz aufhellend und remineralisierend wirken soll, da parallel zur Oxidation die Freisetzung von Kalzium- und Phosphat-Ionen ausgelöst wird. Nicht klar ist, ob die Salze in der verhältnismäßig kurz währenden Einwirkungsdauer einen nennenswerten Zahnaufbau leisten können. US 6,303,104 beschreibt ein oxidationsmittelfreies Zwei-Komponentensystem aus löslichen Kalzium- und Phosphatsalzen, dass auch eine aufhellende Wirkung haben soll. Die Aufhellung soll durch die Zugabe von Natriumgluconat hervorgerufen werden, welches färbende Metallionen (z. B. Eisen) aus dem Zahnschmelz komplexiert. Beim Vermischen der Komponenten ist sofort mit einer Ausfällung des schwerlöslicher Kalziumphosphate zu rechnen und es ist nicht zu erkennen, warum es zu ausgeprägter Remineralisation kommen soll, zumal es sich bei dem Produkt um eine Zahncreme handelt, die nur wenige Minuten in Kontakt mit den Zahnflächen verbleibt. Der entfärbende Effekt reduziert sich zudem auf komplexierbare Metallionen und wird vermutlich nur in den äußersten Schmelzschichten eine Wirkung zeigen. US 6,102,050 beschreibt eine Zahnseide mit Titandioxidpartikeln, der eine aufhellende, remineralisierende und desensibilisierende Wirkung auf die Interdentalflächen zugeschrieben wird. Hier sollen Titandioxidmikropartikel der Größe 0.1-1.5 µm sowohl als mildes Abrasivum wirken, als auch vom Schmelz absorbiert werden, was mit einer aufhellenden Wirkung verbunden sei. Vermutlich können sich die Partikel allenfalls mechanisch in geeignete Hohlräume einlagern, was keiner stabilen Verankerung verspricht und allenfalls einen temporären Effekt haben kann.

Der oben beschriebene Stand der Technik berücksichtigt nicht, dass Biominerale ihre hohe strukturelle Organisation und Stabilität nur erreichen, wenn sie sich in Gegenwart von speziellen Biomolekülen bilden, die Mikro- und Makrostrukturierung vorgeben. WO 2005/027863 beschreibt ein Zahnpflegemittel, dass über reinigende, remineralisierende, desensibilisierende und aufhellende Wirkung verfügen soll. Als aktive Komponente zur Remineralisation und Aufhellung wird ein nanoskaliges Apatit-Gelatine-Komposit vorgeschlagen, das in Gegenwart einer wässrigen Gelatinelösung gefällt wird und daher Polypeptide eingelagert hat. Diesem Material wird zugesprochen, dass es durch sogenannte "Neomineralisation" auf der Zahnoberfläche einen Schutzfilm von dentinähnlicher Struktur bildet, der eine Oberflächenglättung bewirkt und offene Dentintubuli verschließen kann. Dass eine Zahncreme mit einem Gehalt von bevorzugt nur 0.01-2 Gew.% des sogenannten "Nanite" (WO 01/01930) bei einer Einwirkung der aktiven Substanzen liegt von nur einigen Minuten täglich eine solche Wirkung hat, ist als erstaunlich zu bezeichnen. Eine ausgeprägte Mineralabscheidung erscheint eher unwahrscheinlich. Zudem sind diskrete nanoskalige Teilchen farblos, so dass die Einlagerung von Nanite insbesonders in Schichten im Submikrometerbereich nicht zu einer Farbveränderung führen sollte. Eine Mineralabscheidung auf Schmelz ist nicht beschrieben. Es ist ohnehin fraglich, ob und wie sich der geordnete Aufbau der Nanopartikel auf die mikroskopische Ordnung der Prismenstruktur im Schmelz übertragen könnte. Ein kontinuierlicher Zuwachs der Filmdicke bei längerer Anwendung des Pflegemittels wird ebenfalls nicht erwähnt. Zudem ist die poröse, wenig geordnete Struktur von Dentin nicht in der Lage, den Zahn vor korrosiven Angriffen zu schützen. Daher erscheint ein Schutzfilm von dentinartiger Struktur nicht geeignet, einen dauerhaften Schutz zu bieten.

Die Möglichkeit einer kontinuierlich wachsenden FAP-Schicht mit Schmelz- oder dentinähnlicher Struktur bietet die in US 20005220724 und DE1020040545847 beschriebene Technik. Wasserlösliche Phospat- und Fluorid-Salze werden in dem gepufferten Gel A, Kalzium-Ionen im Gel B eingearbeitet. Optional durch eine ionenfreie Schutzschicht getrennt, werden die bei physiologischer Temperatur festen Gelatine-Gele unter Erwärmung nacheinander auf Zahnoberflächen aufgetragen. In Abhängigkeit von den Austauschzyklen der Gele kann eine Zunahme der Schichtstärke beobachtet werden. Die Wachstumsgeschwindigkeiten betragen 0.5-5.0 µm/Tag. Die biologischen Strukturen des Zahnmaterials werden individuell vom Fluorapatit abgebildet, Hohlräume durch offenliegende Dentintubuli werden jedoch nach einigen Austauschzyklen verschlossen. Hinsichtlich einer Anwendung am Menschen erweist es sich als ungünstig, dass die Gele vor der Applikation erwärmt werden müssen. Durch das Aufbringen der zweiten und dritten Gelschicht können darunter liegende, bereits applizierte Gelschichten wieder verflüssigt werden und in unerwünschter Weise mit darüber liegenden Schichten vermischen. Insbesondere kleinere Darreichungsmengen trocknen bei Exposition an der Luft schnell aus und ein Verflüssigen durch Erwärmung ist dann nicht mehr ohne weiteres möglich. Die Methode erlaubt es nur unter Schwierigkeiten, genau definierte Gelmengen auf den Zahn aufzubringen. Ferner tragen die drei bis zu 6 mm dicken Gelschichten stark auf, was bei Schutzsystemen wie Schienen oder Pflastern zu Problemen führt, da hier Platz für große Gelreservoirs geschaffen werden muss.

Die Erfindung betrifft eine Methode zur gezielten Beschichtung der Zähne mit Fluorapatit als zahnfreundliche Alternative zu bisher bekannten oxidativen Bleich-Prozeduren.

Erfindungsgemäß wir ein Kit zur Aufhellung von Zähnen bereitgestellt, aufweisend
**A** eine Gelbildner enthaltende, alkalische, wässrige Natriumfluoridlösung,
**B** ein getrocknetes, in Filmstreifen vorliegendes Gel enthaltend
   - B1 mindestens einen Gelbildner,
   - B2 Phosphat- oder Hydrogenphosphationen,
   - B3 optional mindestens eine Aminosäure,
   - B4 optional Fluorid,
   - B5 optional Glycerin,
      B6 optional eine Carbonsäure oder ein Puffersystem für einen der pH-Werte von 4 bis 7,
**C** ein getrocknetes, in Filmstreifen vorliegendes Gel enthaltend
   - C1 mindestens einen Gelbildner,
   - C2 Calcium-lonen,
   - C3 optional Glycerin.
**B** ist bevorzugt ein vorkonfektionierter, 50-1000 µm dicker Gelfilm.
**C** ist bevorzugt ein vorkonfektionierter 50 µm bis 5 mm dicker Gelfilm.
In C2 sind vorzugsweise 0.05 bis 3 mol pro Liter Gel Calciumionen Ca²⁺ enthalten.
In B2 sind vorzugsweise 0.01 - 2 mol pro Liter Gel eines Phosphats oder Hydrogenphosphats enthalten.
Bei dem Gelbildner handelt es sich bevorzugt um fachübliche Proteinhydrolysate, besonders um Gelatine.
Die Gelatinekonzentration in **B** und **C** beträgt jeweils bevorzugt 1-15 Gew.%, insbesondere 5-10 Gew.%.
Phosphat oder Hydrogenphosphat liegen vorzugsweise als dissoziiertes Na₂HPO₄ (NH₄)₂HPO₄ oder K₂HPO₄ vor.

Die Calciumionen können z.B. als dissoziiertes CaCl₂ vorliegen. Es ist aber auch möglich, schwerer lösliche Ca-Salze - gegebenenfalls zusammen mit leichtlöslichen einzusetzen.

Der Mineralschicht wird gleichzeitig ein protektiver bzw. remineralisierender Effekt hinsichtlich Erosion der Zahnhartsubstanz, Schmerz- und Kariesempfindlichkeit zugeschrieben. Durch gezielte Reaktionsführung und der möglichen Einlagerung bestimmter Weißpigmente lassen sich die optischen Eigenschaften hinsichtlich Farbe, Helligkeit und Opazität der fest haftenden Mineralschicht einstellen.

Um die Vitalität des Zahns nicht zu gefährden und Irritationen des Zahnfleisches zu vermeiden, wird auf den Einsatz konzentrierter Säuren oder Oxidantien vollkommen verzichtet.

Das im Vorliegenden beschriebene Kit kann für eine gezielte, biomimetische Mineralisation von Apatit auf Zahnflächen zu deren Qualitätssteigerung hinsichtlich Oberflächenstruktur eingesetzt werden. Als geeignete Indikation für die hier beschriebene mineralisationsinduzierende Behandlung gilt beispielsweise mit Porosität der Zahnhartsubstanz in Zusammenhang stehenden Hypersensibilitäten. Da bei bestimmten Darreichungsformen bereits Schichten mit einer Stärke von wenigen Mikrometern durch ihre Eigenfarbe eine Aufhellung der Zahnsubstanz bewirken, kann diese Methode auch als verträgliche Alternative zu herkömmlichen Bleichmethoden eingesetzt werden, die darüber hinaus noch einen protektiven Effekt auf die Zähne hat. Die Schichten zeichnen sich durch deutlichen Glanz aus, was für die geringe Rauhigkeit der Mineralabscheidung spricht. Mineralverlust und damit verbundene Glanzverminderung durch intensiven Genuss abrasiver Lebensmittel, können kompensiert werden. Der Prozess hat nichts mit der Neomineralisation bereits vorhandener nanoskaliger Kristallisationskeime gemeinsam, die in Patent WO 2005/027863 beschrieben ist und durch die Ionen im Speichel ausgelöst wird. Vielmehr beruht der im Labor nachgewiesene Effekt der Schichtbildung auf der direkten heterogenen Nukleation von Apatit, bevorzugt fluoridhaltiger Apatit, induziert durch die im Gel angebotenen Ionen. Die gelösten Kalzium-, Phosphat- und Fluorid-lonen kristallisieren dann aus einer gelatinösen Matrix als festhaftende Schicht auf dem Zahn.

Infolge der langen Einwirkdauer der aktiven Mineralien können sich effektiv mit dem Substrat verwachsene, hochgeordnete kristalline Schichten bilden. Ein optimaler Effekt wird bei einer Einwirkungsdauer der erfindungsgemäßen Komponenten ab einem Tag beobachtet. Die minimale Einwirkungsdauer sollte über 2 Stunden, bevorzugt über 8 Stunden liegen. Um die Komponenten sicher am Zahn zu halten, wird eine Abdeckung aus wasserfesten Harzen oder Polymeren vorgeschlagen, wie etwa ein Parodontalverband oder ein lichthärtendes Polymerisat.

Geeignet sind beispielsweise Polysiloxane, Polyacrylate oder Polyamide. Die Aufzählung ist einschließlich aber nicht ausschließlich.

Entsprechend betrifft die Erfindung auch ein Verfahren zur Herstellung eines Kits zur Aufhellung von Zähnen mit den Schritten
**01** Herstellung einer Lösung **A** nach Anspruch 1, indem Natriumfluoridlösung auf pH 12-14 eingestellt und 2-20 Gew.% Gelatine versetzt wird;
**02** Herstellung eines Gels B nach Anspruch 1, indem
   - 0.1 -2 mol/l NaH₂PO₄, 0.01 -2 mol/l NaF, 0,05- 1 mol/l Aminosäure 2-10 Gew. % Essigsäure in Wasser gelöst werden,
   - der pH-Wert mit Lauge auf 3.5 - 6 eingestellt wird,
   - die Lösung mit 10-40 Gew.% Glycerin und 5-20 Gew.% Gelatine zu einem dickflüssigen Gel verarbeitet wird,
   - aus dem noch flüssigen Gel 200 - 400 µm starke Gelfilme ausgestrichen, getrocknet und in passgerechte Stücke oder Streifen geschnitten werden,
**03** Herstellung eines Gels C nach Anspruch 1, indem
   - eine 0,5 bis 3 molare Kalziumchloridlösung angesetzt wird,
   - der pH-Wert wird mit Lauge auf 3.5 -6.0 eingestellt wird,
   - die Lösung mit 10-40 Gew.% Glycerin und 5-20 Gew.% Gelatine zu einem dickflüssigen Gel verarbeitet wird,
   - aus dem noch flüssigen Gel 200 -400 µm starke Gelfilme ausgestrichen, getrocknet und in passgerechte Stücke oder Streifen geschnitten werden.

Des weiteren betrifft die Erfindung ein Verfahren zur kosmetischen Aufhellung von Zähnen, bei dem
- die Zahnoberfläche mit einer Lösung **A** gemäß Anspruch 1 vorbehandelt wird oder eine Lösung **A** gemäß Anspruch 1 aufgetragen und einmassiert wird,
- Gel **B** gemäß Anspruch 1 auf die Zahnoberfläche aufgebracht wird,
- Gel **C** gemäß Anspruch 1 auf Gel **B** aufgebracht wird,
- für eine Einwirkungsdauer von 1-10 h gesorgt wird.

Überraschender Weise erhalten die Schichten ihre aufhellende Wirkung insbesondere, wenn die Ionenkapazität der Gele erhöht und die Auftragsmenge vermindert wird. Um eine höhere Konzentration an Kalzium- und Phosphat-Ionen in löslicher Form im Gel zu speichern, werden der Gelatine bevorzugt eine oder mehrere Aminosäuren zugefügt. Die Aminosäuren wirken durch ihre bindende Wechselwirkung mit den Mineralsalzen wie ein Depot und optimieren deren Verfügbarkeit. Es wird angenommen, dass bei lokaler Verarmung von Kalzium und Phosphat infolge der Mineralisation die Aminosäuren durch Verschiebung des Gleichgewichtes Ionen freisetzen. Grundsätzlich sind alle Aminosäuren geeignet, Kalzium- und Phosphat-Ionen zu binden, da jede als amphoteres Molekül sowohl über eine saure- als auch über eine basische Seitengruppe verfügt. Die optimale Wirksamkeit ist pH-abhängig. Das gilt ebenso für ihre Puffereigenschaft, die sich stabilisierend auf den pH-Verlauf während der Mineralisation auswirken kann. In direkter Umgebung der Mineralisationsfront sind infolge der säurefreisetzenden Apatitbildung besonders diese Aminosäuren aktiv, die über saure Gruppen verfügen und Kalzium freisetzen. Die Verfügbarkeit von Phosphat im Gel A kann bevorzugt durch die Zugabe von Aminosäuren mit zusätzlichen basischen Gruppen erhöht werden. Zur Erhöhung der Löslichkeit sind weiterhin alle Substanzen geeignet, die über Bindungsstellen für Kalzium- und Phosphat-lonen verfügen ohne diese auszufällen oder toxisch auf den menschlichen Organismus wirken. Darunter fallen beispielsweise Vitamine (z.B. Ascorbinsäure), Olygopeptide, Carbonsäuren, insbesondere Fruchtsäuren wie Äpfelsäure, Zitronensäure oder Brenztraubensäure oder Komplexbildner wie EDTA. Erfindungsgemäß wird vorgeschlagen, 1-3 unterschiedliche Gelschichten zu kombinieren, wobei die Reihenfolge festgelegt ist. Soll die Mineralabscheidung mit nur einem Gel erfolgen, wird als flüssige Komponente eine Kalziumphosphat-Lösung eingesetzt, die durch die Einarbeitung in das gelatinöse Medium stabilisiert wird. Die heterogene Nukleation wird durch das Vorbehandlungsmedium ausgelöst. Dieses enthält unter anderem jene Ionensorte, die in der Kalziumphosphat- Lösung im Unterschuss relativ zum stöchiometrischen Verhältnis von Apatit vorhanden ist. Dabei kann es sich sowohl um Phosphat, als auch um Kalzium oder Fluorid handeln. Eine induzierende Wirkung wird überdies durch einen pH-Gradienten zwischen Vorbehandlungsmedium und Gelschicht erzielt. Alternativ wird ein Zwei-Gel-Schichten-Verfahren vorgeschlagen, in dem das Deck-Gel ebenfalls einen induzierenden Effekt auf die Mineralisation hat. Die Verwendung dünner, vorkonfektionierter Gelfilme kompensiert die in US20005220724 beschriebenen Nachteile, die sich aus dem Einsatz warm aufgegossener Gele ergibt. Die Dicke des Phosphat-Gels B oder Kalziumposphat-Gels B* beträgt hierbei 50-1000 µm, bevorzugt 150-500 µm. Die Konzentration der Phosphatsalze beträgt zwischen 0.01 -2 mol/I Gel, bevorzugt 0.08-0.3 mol. Die Fluoridkonzentration beträgt 0-0.3 mol/I, bevorzugt 0-0.05 mol/I. Im Fall von Gel B* beträgt die Kalziumkonzentration 0.0001-0.1 mol. Die Dicke des Kalziumgels C beträgt zwischen 50 µm und 5 mm, bevorzugt 300 bis 600 µm.

DE1020040545847 beschreibt, dass eine Vorbenetzung der Zähne mit 0.05-1 N Natronlauge einen Einfluss auf Morphologie und Wachtumsgeschwindigkeit der Schichten hat, insbesondere, wenn der Lösung Kalziumionen zugefügt werden. Überraschenderweise wurde gefunden. dass eine kalziumfreie, alkalische Fluoridlösung ebenfalls einen wachstumsbeschleunigenden Effekt hat. Die Konzentration an Natriumfluorid liegt zwischen 0.01-1 mol/l. Eine schädigende Wirkung durch zu hohe Fluoriddosen auf den Organismus wird nicht erwartet, da die Mengen der eingesetzten Fluoridlösung pro Zahn zwischen 0.02-0.2 ml liegt. Der Lösung kann Gelatine zugefügt werden, um ihr eine gelige Konsistenz zu verleihen und die Haftung an der Oberfläche zu verbessern. Bevorzugt eingesetzt werden 1-15 Gew.%, besonders bevorzugt 5-10 Gew.%. Der pH-Wert der Lösung liegt zwischen 8-14. Dadurch bilden sich reproduzierbar sehr dünne Filme auf der Zahnoberfläche aus, welche die initiale Mineralisation besonders begünstigen. Je nach Ionengehalt der Gele B bzw. B* und C kann die Vorbehandlungslösung 0.5-3 mol/l Kalzium, oder 0-1 mol/l Phosphat und/oder 0-1 mol/ Fluorid enthalten.

### Vorteile der Erfindung

a) Überraschenderweise wurde festgestellt, dass die Schichten unter bestimmten Bedingungen über einen hohen Glanz verfügen. Es wird davon ausgegangen, dass durch das Ausstreichen dünner Filme die Ordnung der Polypeptidketten im Gelatine-Gel gegenüber dem Auftropfen einer warmen Lösung deutlich erhöht und damit eine bessere Vororientierung gegeben ist. Infolge ist auch die Orientierung des Mineralisats höher.

b) Überraschenderweise wurde festgestellt, dass bereits nach 4-6 Austauschzyklen eine deutliche Aufhellung der Zahnproben zu beobachten ist. Der Aufhellungseffekt intensiviert sich mit zunehmender Anzahl der Austauschzyklen. Auch durch Einbringen von geeigneten Pigmenten in eine oder mehrere der Komponenten **A, B** und **C** (z. B. TiO₂, BaSO₄ usw.) lässt sich ein Aufhellungseffekt bewirken.

c) Im Rahmen der Erfindung wird eine gezielte, biomimetische Mineralisation von Apatit auf Zahnflächen als nebenwirkungsfreie Alternative zu oxidativen Bleichverfahren vorgestellt. Die beschriebene Behandlung kann auch eingesetzt werden, um stumpf gewordenen Zähnen zu neuem Glanz zu verhelfen. Die damit verbundene Verminderung der Oberflächenrauhigkeit und der hohe Fluoridgehalt in den Schichten erhöhen die Kariesresistenz der Zähne.

Die folgenden Beispiele dienen der näheren Erläuterung der Erfindung. Teile- und Prozentangaben beziehen sich, sofern nicht anders angegeben, auf das Gewicht.

### Beispiel 1:

Humanes Zahnmaterial wird in 4 Scheiben mit einer Stärke von je zwei Millimetern gesägt und hochglanzpoliert, um eine möglichst ebene Fläche zu erhalten. Eine Scheibe besteht oberflächlich ausschließlich aus Schmelz, eine ausschließlich aus Dentin und zwei Scheiben enthalten beide Hartgewebstypen. Zwei Scheiben werden für 10 Sekunden mit 25%iger Phosphorsäure behandelt, um die Dentintubuli freizulegen und anschließend intensiv unter fließendem Wasser gewaschen. Die anderen Scheiben bleiben unverändert. Für die alkalische Vorbehandlung wird eine 0.5 molare Natriumfluoridlösung auf pH 14 eingestellt und mit 7.5 Gew.% Gelatine versetzt. Die Zahnscheiben werden mit dieser Lösung eingestrichen und die Feuchtigkeit wird im Anschluss nach 60 Sekunden verblasen.

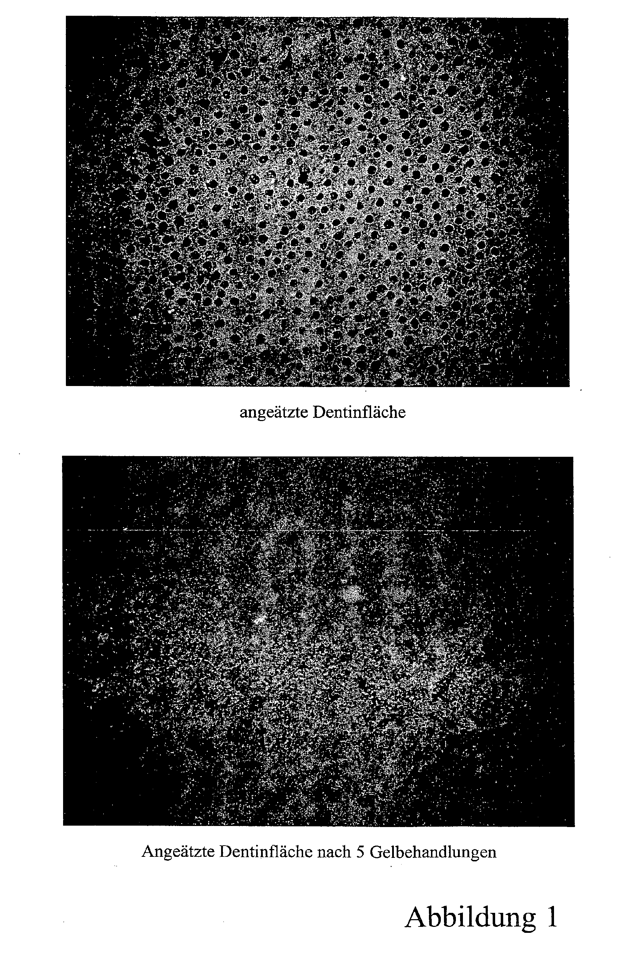

Für das phosphationenhaltige Gel wird eine Lösung hergestellt, die 0.6 mol/I NaH₂PO₄, 0.1 mol/l NaF, 0.3 mol/I Asparagin und 270 ml 2 N Essigsäure enthält. Der pH-Wert wird mit 2 N Natronlauge auf 5.0 eingestellt. 16 ml der Lösung werden mit 6 g Glycerin und 10 g einer 300 Bloom Schweineschwarte-Gelatine unter Erwärmen zu einem dickflüsigen Gel verarbeitet. Mittels Rakel werden aus dem noch flüssigen Gel 300 µm starke Gelfilme ausgestrichen, getrocknet und anschließend in passgerechte Stücke geschnitten. Für das Kalzium-Gel wird eine 1 molare Kalziumchloridlösung angesetzt. Die Weiterverarbeitung entspricht der des Phosphatgels. Mehrere Zahnscheiben werden nun mit je einem Stück Phosphat-Gel und einem Stück Kalziumgel bedeckt. Um die morphologische Änderung der Zahnoberfläche deutlich zu machen, wurde eine Scheibe zuvor zur Hälfte abgeklebt, so dass sie nur hälftig remineralisieren kann. Die Proben werden im Klimaschrank bei 37°C und 95% Luftfeuchtigkeit aufbewahrt, täglich gewaschen und einer erneuten Gelbehandlung unterzogen. Nach wenigen Tauschzyklen zeigen alle Proben einen gleichmäßigen Überzug aus biomimetischem Mineral unabhängig vom vorherigen Ätzen. Abb. 1 zeigt eine lichtmikroskopische Aufnahme eines angeätzten Dentinbereichs vor und nach dem 5. Tausch. Die Dentintubuli sind vollständig verschlossen.

Um den Aufhellungseffekt nachzuweisen, wurden fünf beschichtete Zahnscheiben nach fünf-fachem Gelaustausch mit dem Zwei-Kanalspektralphotometer Spectraflash 600 Plus mit dem CIE L*a*b*-System mit der Blende 3mm X4SAV vermessen. Sofern der Schmelzbereich ausreichte, wurde sowohl eine Dentin- als auch eine Schmelzmessung durchgeführt. Daraus ergaben sich für die Aufhellung ΔL folgende Mittelwerte aus je 3 Einzelmessungen:

**Tabelle 1. ΔL-Werte für Dentin- und Schmelzproben nach fünf-fachem Gelaustausch. Der Mittelwert für Schmelz bei 2.4 (± 1) und bei 3.5(± 0.9) für Dentin.**

| | ΔL |
|---|---|
| P1 Dentin (geätzt) | 4,54 |
| P 2 Schmelz (geätzt) | 2,41 |
| P 2 Dentin (geätzt) | 1,99 |
| P 3 Schmelz (poliert) | 1,61 |
| P 4 Dentin (poliert) | 3,9 |
| P 4 Schmelz (poliert) | 3,22 |

Die Messung zeigt anhand des L-Wertes eine deutliche Zunahme der Helligkeit insbesondere am Dentin.

### Beispiel 2:

Ein Frontzahn wird so präpariert, daß eine ebene Schmelzfläche freigelegt wird, welche poliert wird. Die Probe wird mit einer 7.5 Gew.%igen Gelatinlösung benetzt, deren pH-Wert bei 14 liegt. Dem Gel wurden 0.5 mol/l Natriumfluorid zugefügt. 50 µl der Lösung wurden auf der Zahnfläche einmassiert. Das Aufbringen der Gelfilme entspricht Beispiel 1.

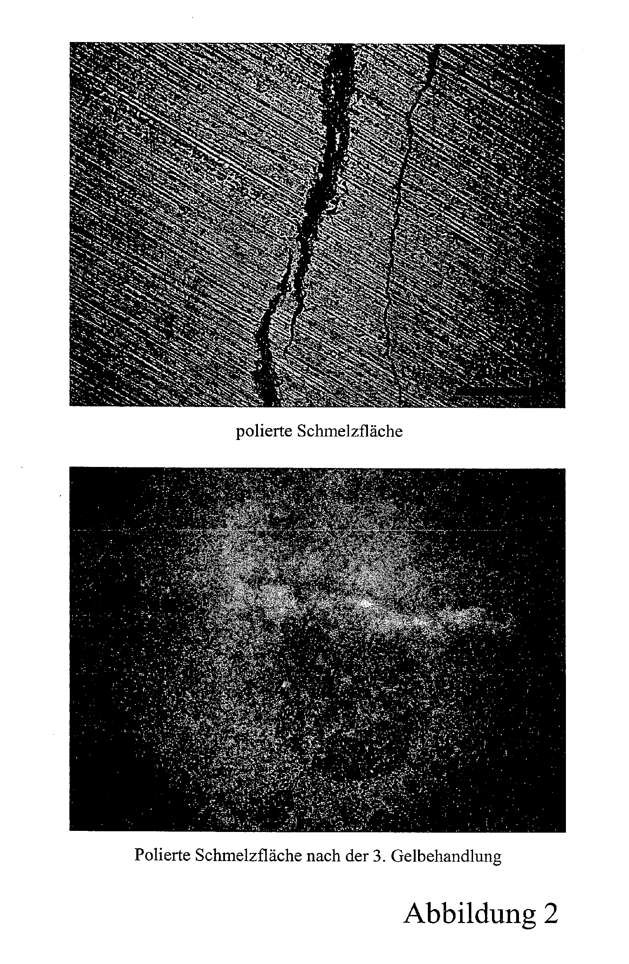

Abb. 2 zeigt eine polierte, aber rissige Oberfläche im Schmelzbereich eines Frontzahns vor und nach dem 3. Tausch. Die Risse sind kaum mehr sichtbar und die Zahnfläche ist gleichmäßig bewachsen.
- Abb. 1: zeigt eine lichtmikroskopische Aufnahme eines Schmelz- und Dentinbereichs vor und nach dem 5. Tausch. Die Dentintubuli sind vollständig verschlossen und eine gewisse Aufhellung ist bereits zu erkennen.
- Abb. 2: zeigt eine polierte, aber rissige Oberfläche im Schmelzbereich eines Incisors vor und nach dem 3. Tausch.

## Patentansprüche

1. Kit zur Aufhellung von Zähnen aufweisend
**A** eine optional Gelbildner enthaltende, alkalische, wässrige Natriumfluoridlösung,
**B** ein getrocknetes, in Filmstreifen vorliegendes Gel enthaltend
• B1 mindestens einen Gelbildner,
• B2 Phosphat- oder Hydrogenphosphationen,
• B3 optional mindestens eine Aminosäure,
• B4 optional Fluorid,
• B5 optional Glycerin,
• B6 optional eine Carbonsäure oder ein Puffersystem für einen der pH-Werte von 4 bis 7.
**C** ein getrocknetes, in Filmstreifen vorliegendes Gel enthaltend
• C1 mindestens einen Gelbildner,
• C2 Calcium-lonen,
• C3 optional Glycerin.

2. Kit gemäß Anspruch 1, bei dem
**B** ein 50-1000 µm dicker Gelfilm ist.

3. Kit gemäß Anspruch 1, bei dem
**C** ein 50 µm bis 5 mm dicker Gelfilm ist

4. Kit gemäß Anspruch 1, bei dem in **C**
C2 0.05 bis 3 mol pro Liter Gel Calciumionen Ca²⁺
enthalten sind.

5. Kit gemäß Anspruch 1, bei dem in **B**
B2 0.01 - 2 mol pro Liter Gel eines Phosphats oder Hydrogenphosphats
enthalten ist.

6. Kit gemäß Anspruch 1, bei dem der Gelbildner Gelatine ist.

7. Kit nach Anspruch 6, wobei die Gelatinekonzentration in **B** und **C** jeweils 1-15 Gew.% beträgt.

8. Kit nach Anspruch 7, wobei die Gelatinekonzentration 5-10 Gew.% beträgt.

9. Verfahren zur Herstellung eines Kits zur Aufhellung von Zähnen mit den Schritten
**01** Herstellung einer Lösung **A** nach Anspruch 1, indem Natriumfluoridlösung auf pH 12-14 eingestellt und optional mit 2-20 Gew.% Gelatine versetzt wird;
**02** Herstellung eines Gels **B** nach Anspruch 1, indem
• 0.1 -2 mol/l NaH₂PO4, 0.01 -2 mol/l NaF, 0,05- 1 mol/l Aminosäure 2-10 Gew % Essigsäure in Wasser gelöst werden,
• der pH-Wert mit Lauge auf 3.5 - 6 eingestellt wird,
• die Lösung mit 10-40 Gew % Glycerin und 5-20 Gew % Gelatine zu einem dickflüssigen Gel verarbeitet wird,
• aus dem noch flüssigen Gel 200 - 400 µm starke Gelfilme ausgestrichen, getrocknet und in passgerechte Stücke oder Streifen geschnitten werden;
**03** Herstellung eines Gels C nach Anspruch 1, indem
• eine 0,5 bis 3 molare Kalziumchloridlösung angesetzt wird,
• der pH-Wert wird mit Lauge auf 3.5 -6.0 eingestellt wird,
• die Lösung mit 10-40 Gew % Glycerin und 5-20 Gew % Gelatine zu einem dickflüssigen Gel verarbeitet wird,
• aus dem noch flüssigen Gel 200 -400 µm starke Gelfilme ausgestrichen, getrocknet und in passgerechte Stücke oder Streifen geschnitten werden.

10. Verfahren zur kosmetischen Aufhellung von Zähnen, bei dem
• die Zahnoberfläche mit einer Lösung **A** gemäß Anspruch 1 vorbehandelt wird oder eine Lösung **A** gemäß Anspruch 1 aufgetragen und einmassiert wird,
• Gel **B** gemäß Anspruch 1 auf die Zahnoberfläche aufgebracht wird,
• Gel C gemäß Anspruch 1 auf Gel **B** aufgebracht wird,
• für eine Einwirkungsdauer von 2-10 h gesorgt wird.

11. Kit oder Verfahren nach einem der vorstehenden Ansprüche, wobei das Phosphat oder Hydrogenphosphat als dissoziiertes Na₂HPO₄ (NH₄)₂HPO₄ oder K₂HPO₄ vorliegt.

12. Kit oder Verfahren nach einem der vorstehenden Ansprüche, wobei die Calciumionen als dissoziiertes CaCl₂ vorliegen.
